# EUROPEAN PATENT SPECIFICATION

(11) **EP 2 956 184 B1**
(45) Date of publication and mention of the grant of the patent: **21.06.2017**
(21) Application number: 14724509.6
(22) Date of filing: 14.02.2014
(51) Int. Cl.: A61L 27/36

(54) **SILK BASED POROUS SCAFFOLD AND A PROCESS FOR THE PREPARATION THEREOF**
PORÖSES GERÜST AUF BASIS VON SEIDE UND VERFAHREN ZUR HERSTELLUNG DAVON
ÉCHAFAUDAGES POREUX À BASE DE SOIE ET LEUR PROCÉDÉ DE PRÉPARATION

(30) Priority: 14.02.2013 IN DE04212013
(43) Date of publication of application: 23.12.2015
(73) Proprietor: Council of Scientific and Industrial Research, New Delhi 110 001 (IN)
(72) Inventor: NISAL, Anuya, Pune 411008 Maharashtra (IN); VENUGOPALAN, Premnath, Pune 411008 Maharashtra (IN); SINHA, Nairiti, Mumbai 400019 Maharashtra (IN)
(74) Representative: Graf von Stosch, Andreas
(86) International application number: PCT/IN2014/000094
(87) International publication number: WO 2014/125505

(56) References cited:
- US-A1- 2008 038 236
- B. B. MANDAL ET AL: "High-strength silk protein scaffolds for bone repair", PROCEEDINGS OF THE NATIONAL ACADEMY OF SCIENCES, vol. 109, no. 20, 15 May 2012 (2012-05-15) , pages 7699-7704, XP055127736, ISSN: 0027-8424, DOI: 10.1073/pnas.1119474109
- JING QU: "Preparation of Silk Fibroin Microspheres and Its Cytocompatibility", JOURNAL OF BIOMATERIALS AND NANOBIOTECHNOLOGY, vol. 04, no. 01, 1 January 2013 (2013-01-01), pages 84-90, XP055127749, ISSN: 2158-7027, DOI: 10.4236/jbnb.2013.41011

## Description

### FIELD OF THE INVENTION

The invention relates to a silk based porous scaffold and a process for the preparation thereof. Particularly, the present invention provides a porous scaffold prepared by fusing silk fibroin particles using a dilute silk fibroin solution. The invention further relates to the process of preparation of the porous scaffold of silk fibroin devoid of any other non-biocompatible chemicals and involving fusion of silk fibroin particles.

### BACKGROUND AND PRIOR ART

Silk Fibroin has been extensively used to prepare porous scaffold for tissue engineering applications on account of its excellent biocompatibility, biodegradability and excellent thermo-mechanical properties. Different methods have been reported for the preparation of these porous scaffolds.

However, porous scaffolds reported in the prior art have certain limitations. For example, the article titled "Preparation of Porous Scaffolds from Silk Fibroin Extracted from the Silk Gland of Bombyx mori (B. mori)" published in Int. J. Mol Sci. 2012;13(6):7762-75, dated Jun 21, 2012. by Yang M et.al discloses a method to prepare porous silk scaffolds by extracting aqueous silk fibroin solution (ASF) from silk gland of 7-day-old fifth instar larvae of *Bombyx mori* (B. mori). The fabrication of porous scaffolds from ASF was achieved by using the freeze-drying method. The scaffolds have porosities greater than 80% and a compressive modulus of 6.9MPa. However, the pore size, shape and pore size distribution cannot be effectively controlled. It has been observed by authors O'brien *et al.* (O'Brien F J, Harley BA, Yannas IV, Gibson.in the article 'The effect of pore size on cell adhesion in collagen-GAG scaffolds' published in . Biomaterials 2005; 26: 433-41) that the pore size critically affects adhesion and proliferation of cells. Also, preparing scaffolds with lower porosities (~ 50%) cannot be done by this process. Low porosities may be desirable when mechanical integrity of the scaffold in the biomed application is crucial for example in use for bone tissue applications. There is also a limitation on the maximum mechanical property that can be achieved using the method described above.

An article titled "A Preliminary In Vivo Study on the Histocompatibility of Silk Fibroin" by Lu Yan et al published in Biomaterials - Physics and Chemistry, discloses a process for preparation of scaffold, wherein the process comprises "dissolving the raw silk after degumming into aqueous solution of LiBr (lithium bromide) by adjusting the concentration up to 20%, followed by addition of n-butyl alcohol in a ratio of 2: 1. The mixed solution was added into self-made mound after low-speed agitation, white porous scaffold was obtained after freeze-drying. The average pore size of the scaffold was between 10 and 20um.

US 7842780 discloses a process comprising the steps of: (a) forming a silk fibroin solution comprising silk fibroin in an aqueous salt solution; (b) removing the salt and water from the fibroin solution to form a silk fibroin substance; (c) forming a polymer solution comprising about 5 to 35% by weight of the silk fibroin substance in a solvent; (d) contacting the polymer solution with water-soluble non-toxic particles that are insoluble in organic solvents and have a diameter between about 50 and about 1000 microns; (e) placing the polymer solution into a form; (f) removing the solvent from the polymer; (g) contacting said polymer solution with an effective amount of an agent to induce [beta]-sheet structure and insolubility in aqueous solution; (h) leaching said polymer with a solvent in which said particles are soluble and polymer is insoluble to remove said particles from said polymer; and (i) drying said polymer.

Both the processes described above require addition and correspondingly removal of chemicals during the scaffold making process. This not only adds up to the manufacturing cost of the scaffold, but it could also result in decreased biocompatibility even if trace amounts of chemicals are present. Also, the microstructure (silk protein conformation) in the scaffold is uniform throughout. If a distribution of the microstructure can be achieved using right processing parameters it can then be used as a lever to control the degradation time of the scaffold. This control over silk protein conformation is restricted using above processing methods.

From the above examples it can be seen that several different processes such as emulsion freeze drying, phase separation, etc. and many more such as porogen leaching, 3D printing have been used to produce silk based scaffolds. (Zhang 2009 - Zhang, Q. et al. "Silk Fibroin based porous materials", Materials, 2, Pg. 2276-2295. Doi. 10.3390/ma2042276). But presently there is no mechanism to include multifunctionality in the scaffolds. In typical tissue engineering applications there is a need to include functionalities other than just cell growth and proliferation. For example, the prior art technologies cannot be used as a drug delivery platform or these methods do not have the flexibility to incorporate other biomolecules like growth factors.

The prior art related issues of silk fibroin porous scaffold described above have been overcome by the present invention. This is achieved by a novel process of fusing together spherical silk fibroin particles which results in controlled pore size and porosity. The method of preparation of silk fibroin particles by using a syringe pump, freezing and lyophilizing them and subsequently annealing them is similar to the prior art (" Silk fibroin spheres as a platform for controlled drug delivery" by Esther Wenk, Anne J. in Journal of Controlled Release 132 (2008) 26-34; Preparation of Silk Fibroin Microspheres and Its Cytocompatibility" by Jing Qu, Lu Wang1 et. al in Journal of Biomaterials and Nanobiotechnology, 2013, 4, 84-90; "Regulation of Silk Material Structure by Temperature-Controlled Water Vapor Annealing" by Xiao Hu, Karen Shmelev et. al in Biomacromolecules. 2011 May 9; 12(5): 1686-1696, doi: 10.1021/bm200062a). However these particles were typically used in drug delivery application or for cytocompatibility studies. B.B.Mandal et al. "High-strength silk protein scaffolds for bone repair", Proceedings of the National Academy of Sciences, 109(20), 15-05-2012, 7699-7704 [XP055127736] disclose the preparartion of microfiber reinforced silk scaffolds. We report a process where we fuse these silk fibroin particles using dilute silk fibroin solutions to form a 3D porous structure. This results in a scaffold that has bulk porosities in the range of 40-70% and typical interparticle pore sizes of 100-500microns. This type of pore architecture is proven to be successful for various implants (www.biopore.in). These scaffolds have excellent mechanical properties (Dry compression modulus ~15-30MPa) as against those reported for most other silk fibroin scaffolds (∼0.1-1 MPa) without any reinforcement. The degradation of these scaffolds can be controlled by controlling the silk fibroin microstructure. All other reported scaffolds have uniform microstructure while we demonstrate that the surface crystallinity is different from the core of the particle and hence there is a range of microstructures possible. The process is also flexible enough to incorporate various additives such as fillers, biomolecules such as drugs, growth factors, proteins, etc. Also, the process does not involve use of any non-biocompatible materials during the making of the scaffold.

### OBJECT OF INVENTION

Main objective of the present invention is to provide a silk based porous scaffold and a process for the preparation thereof.

Another objective of the present invention is to provide a porous scaffold prepared by fusing silk fibroin particles using a dilute silk fibroin solution.

Another objective of the present invention is to provide a porous scaffold comprising silk fibroin with desired pore size and pore size distribution and porosity for cell adhesion and migration.

Yet another objective of the invention is to provide a process for development of scaffolds with excellent mechanical properties and tunable degradation time.

Yet another object of the invention is to provide a platform technology flexible enough for incorporation of different biomolecules such as drugs, growth factors, etc. depending on the end application.

Yet another object of the invention is to provide a simple, cost effective process for preparation of a porous scaffold comprising silk fibroin.

### SUMMARY OF INVENTION

1. Accordingly, the present invention provides a silk fibroin 3D scaffold, which is prepared by the process as described in claim 1, having amorphous, partially crystalline or crystalline microstructure comprising regenerated silk fibroin having silk fibroin particles in the range of 0.1-3 mm and intra particle pore size in the range of 1 - 20µm characterized in that said scaffolds have a modulus of ~ 1-14.7MPa in dry state and ~ 0.1- 0.5 MPa in wet state and the pore size in the range of 100-500µm with interconnections and porosity in the range of 40-70 %.

In an embodiment of the present invention silk fibroin scaffold is useful for the incorporation of drug, protein, growth factor or a filler for biomedical application.

In one embodiment of the present invention degradation of scaffold is tuned by controlling by conformation of silk fibroin protein.

The present invention provides a process for the preparation of silk fibroin scaffolds, devoid of any additives, involving fusion of silk fibroin particles comprising;
a. providing silk fibroin particles from regenerated silk fibroin(RSF) solution by adding RSF solution into liquid nitrogen dropwise and further freezing, lyophilizing and annealing to introduce beta sheet in the presence of a solvent by known method;
b. fusing the particles of step (a) with dilute RSF solution in a mold for compressing and placing mold in a vacuum oven for a period ranging between 1-4 hours at temperature ranging between 40-60°C for drying to form a scaffold.

In another embodiment of the present invention the particles of step (a) before freezing and lyophilizing are amorphous or semi crystalline.

Still in another embodiment of the present invention the particles of step (a) are freezed at a temperature in the range -20 to -80 °C and lyophilized at a temperature in the range of - 48 to -55 °C.

Still in another embodiment of the present invention the solvent for annealing in step (a) is selected from water or methanol.

Still in another embodiment of the present invention concentration of dilute RSF solution used in step (b) is in the range of 1-5wt%.

Still in another embodiment of the present invention the particle size is in the range of 0.1-3mm and the pore size in the range of 100-500µm with interconnections.

Still in another embodiment of the present invention the scaffolds have a modulus of ~ 1-14.7MPa in dry state and ~ 0.1-0.5 MPa in wet state.

### BRIEF DESCRIPTION OF THE DRAWINGS:

**Figure 1**: (a) Schematic diagram for preparation of silk particles (b) Silk particles as obtained
**Figure 2**: Schematic diagram for water annealing process
**Figure 3**: The IR graph comparison of the amorphous silk fibroin formed in step (a) of the process and the IR graph after water annealing as described in step (c) of the process of the invention.
**Figure 4**: Scaffold as formed; Scanning electron micrograph showing pore sizes of 100µm and more.
**Figure 5** Image of particles as described by step (a) in the process and annealed using methanol or water process as described in step (c)
**Figure 6** Dynamic Mechanical properties of scaffolds prepared in Example 6 using 11wt% RSF solution in both wet and dry state
**Figure 7** Thermo-gravimetric analyses on particles prepared in Example 1 and Example 9
**Figure 8** Release of cephalexin drug from water-annealed particles as a function of time.
**Figure 9****:** FTIR spectrum deconvolution using 12 peaks.

### DETAILED DESCRIPTION OF THE INVENTION

The present invention provides a novel, simple, cost effective process for synthesis of silk fibroin scaffolds (A), devoid of any non-biocompatible chemicals by fusing together silk fibroin particles. The porous silk fibroin scaffolds possess excellent mechanical modulus, have tunable degradation properties and can incorporate biological and sensitive drugs/formulations.

The process for the synthesis comprises:
(a) Obtaining silk fibroin particles from regenerated silk fibroin (RSF) solution;
(b) Freezing and lyophilizing the particles of step (a);
(c) Annealing the particles of step (b) to introduce beta sheet/ cross-linking formation; and
(d) Fusing the particles of step (c) using dilute RSF solutions to form a scaffold.

The silk fibroin particles were obtained by dropping the regenerated silk fibroin (RSF) solution in liquid nitrogen bath, freezing and consequently lyophilizing them. The particles may have amorphous or partially crystalline microstructure. More preferably the RSF solution was dropped in to the nitrogen bath using a syringe without any voltage system thus making the process cost-effective.

Accordingly, the process steps include:
a. Dropping a controlled amount of regenerated silk fibroin (RSF) solution in the concentration range of 1-25 w/w % using a syringe fitted with a needle with an internal diameter of 0.1-0.4mm into liquid nitrogen to obtain particles;
b. Freezing and lyophilizing the particles of step (a);
c. Annealing the particles of step (b) using a polar solvent to introduce beta sheet/ cross-linking formation;. and
d. Fusing the particles of step (c) using dilute 3-wt % RSF solution to form a scaffold.

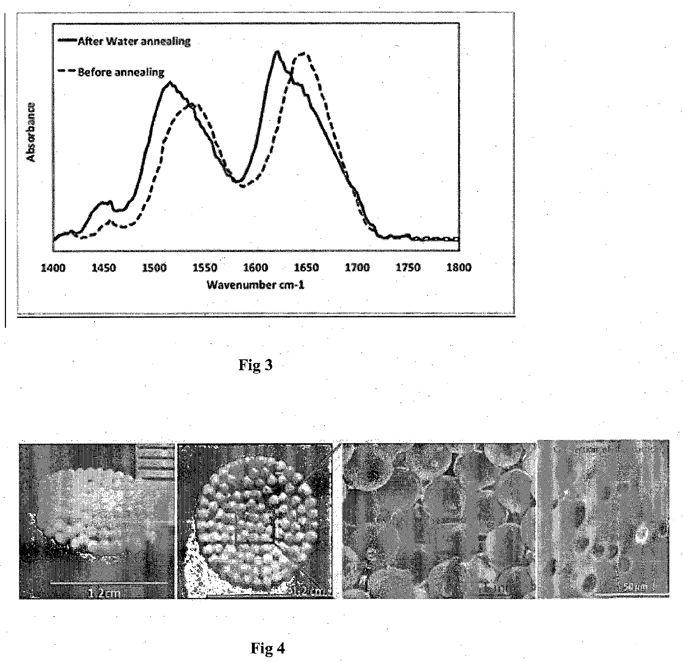

The amount of regenerated silk fibroin (RSF) solution used varied in the range of 1-25 w/w%. The regenerated silk fibroin solution was dropped into liquid nitrogen by means of a syringe, with a flow rate of 0.1 - 1ml/minute. The particles were freezed at a temperature in the range -20 to -80 deg. C for 14-17 hours and lyophilized at -50 deg C for 7-9 hours. The particles as obtained in step (b) of the process yielded amorphous silk fibroin particles, as observed in the IR spectrum **(****Figure 3****)**. The particle size varied between 1 mm and 2.5 mm and the amorphous particles were water-soluble.

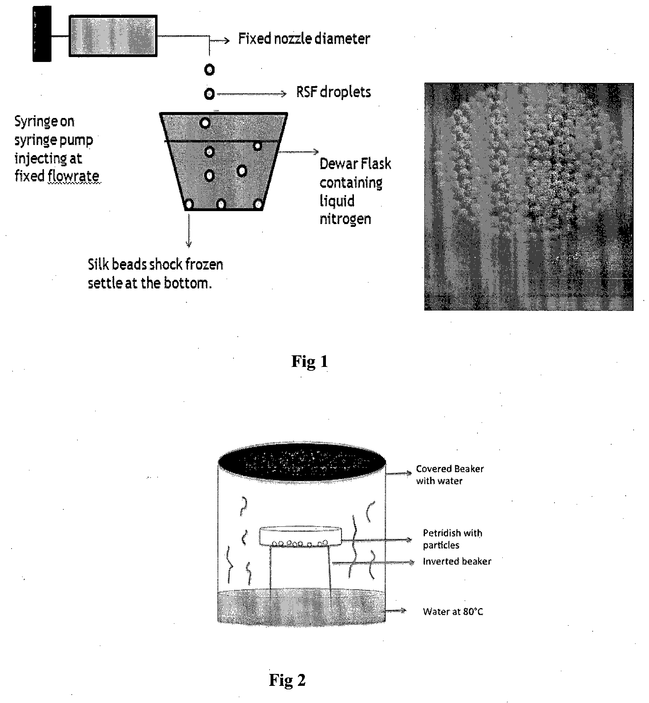

The amorphous particles obtained were annealed by using water vapor as per the process described below. Water annealing was performed to introduce beta sheet formation and was carried out by spreading out the particles in a petri dish and subjecting them to water vapor treatment for an hour as shown herein in **Figure 2**. In an aspect, the crystallinity of the scaffold was determined by controlling the temperature of water and time for annealing. The temperature of the water in the lower petri dish was maintained at 80 deg. C. The average particle diameter of the annealed particles was observed to be 1.1 mm with a SD (standard deviation) of 0.108. The average percentage shrinkage was calculated to be approximately 52.8% by volume. The annealed particles of silk fibroin were water insoluble. Step (c) of the process led to the formation of beta sheet and the surface of the particle had higher beta sheet content as compared to the core as enlisted in **Table 2** below.

Alternately, amorphous particles obtained were annealed using methanol wherein the particles were completely immersed in 100% methanol for 1 h at room temperature. Annealing resulted in particle collapse resulting in a decrease in intra-particle porosity. The particles were oval in shape and had an average aspect ratio of 1.38. Annealing also changed the conformation of silk fibroin protein from predominantly amorphous random coil to crystalline antiparallel beta sheet structures. An annealed particle did not readily disintegrate into 1ml water.

The water-annealed particles were further fused together by first wetting them with a 3wt% dilute RSF solution in water. These particles were then filled in a mold and lightly compressed. The mold was then placed in a vacuum oven for 2 hours at 60 deg C for drying. The scaffold so formed was removed from the mold. The scaffold formed by the process is as depicted in **Figure 4**.

The scaffolds of silk fibroin have porosity in the range of 40-70%. The pore sizes are in the range of 100-500µm. The intra particle pore sizes varied from 1-20 µm.

The porous silk fibroin scaffold prepared by the process which is devoid of additives has porosity in the range of 40-70% particle size in the range of 0.1-3 mm and the pore size in the range of 100-500µm with interconnections.

The scaffolds have a modulus of 1-30MPa in dry state and ~ 0.5 - 2.5MPa in wet state and were observed to have comparable enzymatic degradation at different annealing protocols viz. water annealing and methanol annealing.

The porous silk fibroin scaffold possessing excellent mechanical modulus gave the added flexibility of incorporating other biological and sensitive drugs/formulations into the particles.

The present invention provides a composition comprising an active ingredient selected from a drug, protein, a filler or such like and a porous scaffold (A) of fused particles comprising silk fibroin.

The porous silk fibroin scaffold of the present invention may be used for drug, protein delivery and various other applications such as scaffolds in tissue engineering.
The novelty of the present invention lies in fusing the silk fibroin micro-particles using dilute Regenerated Silk Fibroin solutions. The process of making and annealing these particles is known in the prior art. However, these particles have been discussed for drug delivery kind of applications and cytocompatibility studies. No prior art discusses fusing of these particles to form a 3D scaffold. Further, the process of fusing these particles results in scaffold with unique pore sizes and pore size distributions (100-500microns and intraparticle pores 1-20microns). The bulk porosity of these scaffolds is also lower (40-70%) as compared to those reported in the prior art (typically greater than 80%). This type of pore structure is proven to beneficial for implants (www.biopore.in). These scaffolds have excellent mechanical properties (Dry compression modulus ∼15-30MPa) as against those reported for most other silk fibroin scaffolds (~0.1-1 MPa) without any reinforcement. The degradation of these scaffolds can be controlled by controlling the silk fibroin microstructure. All other reported scaffolds have uniform microstructure while the present invention demonstrates that the surface crystallinity is different from the core of the particle and hence there is a range of microstructures possible. The process is also flexible enough to incorporate various additives such as fillers, biomolecules such as drugs, growth factors, proteins, etc. Additionally the process does not involve use of any non-biocompatible matreial while making of scaffold.
The regenerated silk fibroin is obtained from silk fibroin selected from the group consisting of silks from silkworms, silks from spiders, silks from genetically engineered cells, silks from transgenic plants and animals, silks from cultured cells, native silk, silk from cloned full or partial sequences of native silk genes, and silk from synthetic genes encoding silk or silk-like sequences. Preferably fibroin is obtained from Bombyx mori silkworms by the process known in the art.

The silk scaffolds are more desirable in terms of:
- Controlled pore size and shape and porosities
- Excellent mechanical properties.
- Controlled degradability
- Does not require any non-biocompatible chemicals except for preparation of RSF solutions
- Protocol is flexible to incorporate various formulations (eg. other biological molecules, fillers, etc)

Following examples are given by way of illustration therefore should not be construed to limit the scope of the invention.

### Example 1: Preparation of silk fibroin particles

Pure bivoltine cocoons were procured from Central Sericultural Research and training Institute, Sriramapura, Mysore, Karnataka. The cocoons were boiled in 0.5% solution of sodium bicarbonate twice for 30 mins to remove the sericin protein. 5gm of the silk fibroin (SF) protein was dissolved in 50 ml of 9.3M Lithium Bromide (LiBr) solution at 60°C for 4 hrs. The SF-LiBr solution was then dialysed extensively against water for 48h to ensure complete removal of the liBr salt. Water was changed 5 times during dialysis. The SF solution in water so obtained is called as Regenerated Silk Fibroin (RSF) solution. This solution was then dialysed against 26wt% 20000 g/mol Polyethylene glycol solution to obtain 11wt% RSF solution. A 6ml syringe was fitted with a 26G needle. The injection speed of the syringe pump was fixed at 0.1 ml / min. The drops of RSF were allowed to fall into a LN2 (liquid nitrogen) dewar. These particles were further freezed at -80°C in a freezer for 15 hrs and then lyophilized at -50°C for 8hrs. The resulting particles were amorphous (**Figure 3**), with an average particle size of 2.03mm and a standard deviation (SD) of 0.188 mm, after particle size analysis of 105 particles using ImageJ software. The Minimum and maximum sizes were recorded as 1.338mm and 2.530mm. These results have been summarized in **Table 1**. When a single bead was added to 1 ml water at room temperature, a clear solution was obtained, indicating complete water solubility of particles.

This indicated that the particles had silk fibroin protein in random coil conformation and this is indicated by the crystallinity index calculated from FTIR measurements.

**Table 1: Statistical data after image analysis done on particles prepared using process in Error! Reference source not found.**

| | |
|---|---|
| Mean particle diameter | 2.03 |
| % Standard deviation | 9.26 |
| d10 | 1.83 |
| d50 | 2.01 |
| d90 | 2.29 |
| d10/d90 | 0.80 |
| Crystallinity index*(Std. dev.) | 0.74 (±0.06) |

Crystallinity Index = Ratio of areas of three crystalline beta sheet peaks (1616-1621, 1622-1627, 1628 -1637cm⁻¹) to ratio of random coil peaks (1638-1646 and 1647-1655cm⁻¹) as obtained after peak Deconvolution and curve fitting of Infrared spectra in amide I region.

### Example 2: Process of water annealing the particles

The particles as obtained in example 1 were then water annealed to introduce beta sheet formation. The untreated particles were spread out in a petri dish and subjected to water vapor treatment for an hour as shown herein (Fig. 2). The temperature of the water in the lower petri dish was maintained at 80 °C. Annealing resulted in particle collapse. It also changed the conformation of silk fibroin protein from predominantly amorphous random coil to crystalline antiparallel beta sheet structures. An annealed particle did not readily disintegrate into 1ml water.

The diameter of the water-annealed particles was measured as described in example 1. An average particle diameter was found to be 1.072mm with a SD of 0.108. The percentage shrinkage was calculated to be approximately 52.8% by volume. Refer **Table 3**.

These beads retained more than 90% of the weight even after immersion in water for one hour. The microstructure of these beads was characterized using Fourier Transform Infrared spectroscopy in the Attenuated Total Reflectance mode. The ATR data was deconvoluted using a Peak fit software (v 4.12) and the crystallinity index on the surface and in the bulk of the particle were determined. The results have been summarized in **Table 2.**

**Table 2: Microstructure of the silk fibroin particles**

| | Before annealing and core of particle | Before annealing and surface of particle | After annealing and core of particle | After annealing and surface of particle |
|---|---|---|---|---|
| Crystallinity index | 0.80 | 0.50 | 1.08 | 1.16 |

The above results suggest that water annealing results in beta sheet formation and the surface of the particle has higher beta sheet content as compared to the core after annealing.

### Example 3. Process of methanol annealing the particles

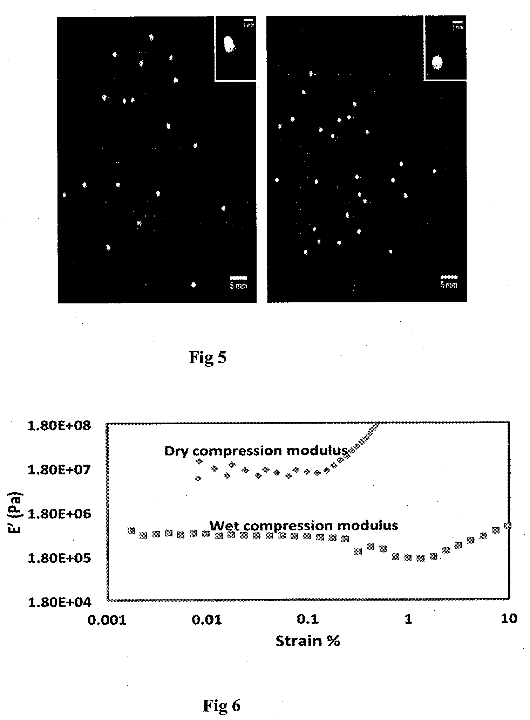

The particles were prepared as per the protocol described in Example 1. These particles were completely immersed in 100% methanol for 1 h at room temperature (25°C). Annealing resulted in particle collapse resulting in a decrease in intra-particle porosity. The particles were also oval in shape and had an average aspect ratio of 1.38. See **Table 4** for details. An average value of the long and short diameters has been reported in **Figure 5** as the effective diameter. Annealing also changed the conformation of silk fibroin protein from predominantly amorphous random coil to crystalline antiparallel beta sheet structures. An annealed particle did not readily disintegrate into 1ml water.

**Table 3: Statistical data after image analysis done on particles annealed with methanol and water process.**

| | Methanol annealing | Water annealing |
|---|---|---|
| Mean effective particle diameter (mm) | 1.21* | 1.06 |
| % Standard deviation | 6.21 | 8.50 |
| d10 | 1.09 | 0.93 |
| d50 | 1.20 | 1.05 |
| d90 | 1.27 | 1.15 |
| d10/d90 | 0.85 | 0.81 |
| Aspect ratio | 1.38 | 1.01 |
| Crystallinity index (Std. dev) | 1.30 (±0.05) | 1.11 (±0.05) |

| | | |
|---|---|---|
| *Effective diameter = average diameter calculated after measuring the major and minor axis of methanol annealed particles | | |

### Example 4: Preparation of scaffolds

The water-annealed particles of example 2 were then fused together by first wetting them with a 3wt% dilute RSF solution in water as in example 1. 20mg of silk particles and 60µl of RSF solution was used for the same. These particles were then filled in a mold and compressed. The mold was then placed in a vacuum oven for 2 hours at 60°C for drying. A typical scaffold formed is as shown in Figure 4. The interparticle pore size was found to be in the range of 100-500 microns with an average pore size of 273 microns.

### Example 5: Change in particle size

Three different RSF solutions of concentrations 3wt%, 6wt% and 11wt% were used to prepare the particles as per the process described in **Example 1.** An image analysis on these particles was done to estimate the particle size and size distributions. The data is summarized in Table **4** below. The particles were water annealed as per the protocol described in **Example 2** and image analysis results for the same is also included in **4**. It was observed that the diameter of the particle did not change significantly with concentration before annealing. However, after annealing a range of particle sizes were produced by merely changing the RSF concentration used.

**Table 4: Image analysis on particles prepared using RSF solutions of 3wt%, 6 wt% and 11wt% RSF solutions both before and after water annealing.**

| | Before annealing | | | After water annealing | | |
|---|---|---|---|---|---|---|
| RSF solution concentration | 3wt% | 6wt% | 11wt% | 3wt% | 6wt% | 11wt% |
| Mean particle diameter(mm) | 2.21 | 2.01 | 2.03 | 0.68 | 0.95 | 1.06 |
| % Standard deviation | 6.57 | 4.46 | 9.26 | 11.72 | 7.58 | 8.50 |
| d10 | 2.11 | 1.88 | 1.83 | 0.57 | 0.86 | 0.93 |
| d50 | 2.20 | 1.99 | 2.01 | 0.68 | 0.96 | 1.05 |
| d90 | 2.27 | 2.13 | 2.29 | 0.75 | 1.04 | 1.15 |
| d10/d90 | 0.85 | 0.88 | 0.80 | 0.77 | 0.83 | 0.81 |
| Crystallinity index | 0.85 (±0.01) | 0.91 (±0.03) | 0.74 (±0.06) | 1.31 (±0.04) | 1.10 (±0.03) | 1.11 (±0.05) |

### Example 6. Measurement of mechanical properties of scaffolds prepared

The dynamic mechanical properties of the scaffold were measured in both dry and wet state in an amplitude sweep experiment done on the RSA III model (TA instruments) of Dynamic Mechanical Analyzer. The dry modulus was done on the scaffold prepared in Example 4 using 11wt RSF solution. The wet modulus was done on the scaffold prepared in Example 4 after allowing the scaffold to swell in DI water for 2 hrs. The results are summarized in **Figure 6**. The scaffolds have a storage modulus of ∼ 14.7MPa in dry state and 0.51MPa in wet state (Values obtained by averaging the modulus in the range of 0.01 to 0.1% strain).

### Example 7: Degradation with respect to annealing protocol

The particles prepared via annealing protocols described in Example 2 and Example 3 were used to prepare scaffolds as per the protocol described in Example 4. These samples were evaluated for enzymatic degradation in Protease XIV solution. The scaffolds were incubated in 1U/ml enzyme solution for 4 days under sterile conditions and their weight loss was monitored after 4 days degradation. 20mg of Scaffold was incubated in 5ml of enzyme solution. The results are tabulated in **Table 5**. The results suggest that although the annealing protocols are different, both the scaffolds degrade at comparable rates.

**Table 5: Comparison of enzymatic degradation of scaffolds with different annealing protocols for particles**

| | Methanol annealed | Water annealed |
|---|---|---|
| Weight % retained for scaffold after 4 days enzymatic degradation | 64.0 | 60.5 |

### Example 8: Preparation of particles with fillers

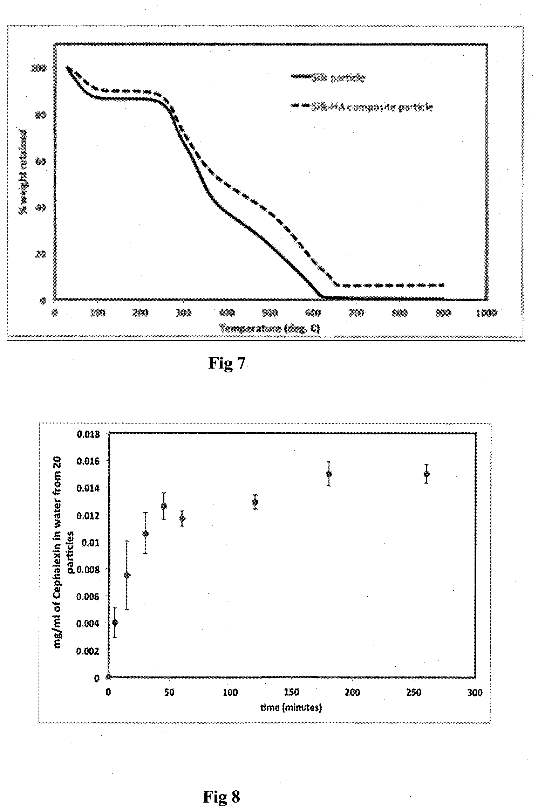

4.5mg of nano-Hydroxyapatite (HAP) powder was blended with 1 ml of 3wt% RSF solution. The composite solution was used to prepare silk fibroin particles as per the protocol described in **example1.** A needle of 20G was used to prevent choking and the solution was constantly stirred using a magnetic needle to ensure uniform mixing of HAP powder. The particles prepared were characterized and the results summarized in **Figure 7** and Table 7. The thermo-gravimetric analysis suggests that about 6.3 % of HAP has been successfully incorporated into the silk fibroin-HAP composite particles. The particles prepared in **example1** do not retain any weight beyond 620°C.

**Table 7: Statistical data after image analysis done on Silk Fibroin - HAP particles prepared using process in Example 9.**

| | |
|---|---|
| Mean particle diameter | 2.72 |
| % Standard deviation | 10.12 |
| d10 | 2.47 |
| d50 | 2.61 |
| d90 | 3.10 |
| d10/d10 | 0.80 |
| Crystallinity index** | 0.93 (±0.01) |

### Example 9: Incorporation of drugs

Cephalexin, a common antibiotic drug, was used for this experiment. 10mg/m of cephalexin stock solution was prepared and 240µl of this was added to 6ml of 3wt% RSF solution. This SF-cephalexin solution was used to prepare silk fibroin particles using the protocol described in **example1** and the particles were later water annealed as in **example 2**. The release of the drug from the particles at RT in water was monitored by measuring the absorbance as a function of time in a UV spectrophotometer at 262nm⁻¹. A calibration curve was used to prepare a plot of concentration drug released versus the time as shown in **Figure 8**. The example clearly demonstrates the flexibility of this method to incorporate a variety of biomolecules depending on the biomedical application under consideration.

### Example 10: FTIR analysis on Silk fibroin

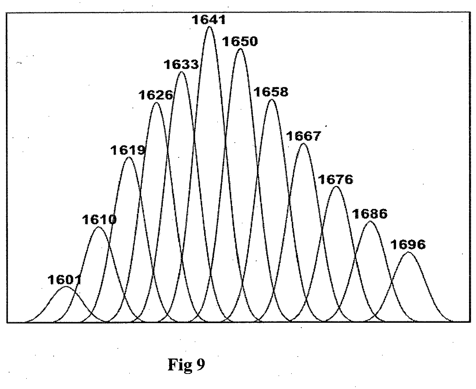

The FTIR spectrum in the amide I region (1580-1720cm⁻¹) was deconvoluted using the Peakfit v 4.12 software. The spectrum was corrected for baseline using a linear two-point method. A second derivative method was used to identify the peaks and the spectrum was smoothened till 12 peaks (1595-1605, 1605-1615, 1616-1621, 1622-1627, 1628-1637, 1638-1646, 1647-1655, 1656-1662, 1663-1670, 1671-1685, 1686-1696, 16,96-1703cm⁻¹) could be fit. These peak positions have been defined by Hu et al. (Hu et al., 2006). A Gausssian fit with fixed peak width was used for automatic curve fitting and the spectrum was deconvoluted into 12 peaks as shown in **Figure 9**. A crystallinity index is defined as the ratio of sum of the absolute areas under the three beta sheet peaks (1616-1621, 1622-1627, 1628-1637 cm-1) to the sum of absolute areas under the two random coil and alpha helix peaks (1638-1646, 1647-1655, 1656-1662 cm-1). Thus for the example shown, the surface of the particles has a crystallinity index of 0.74.

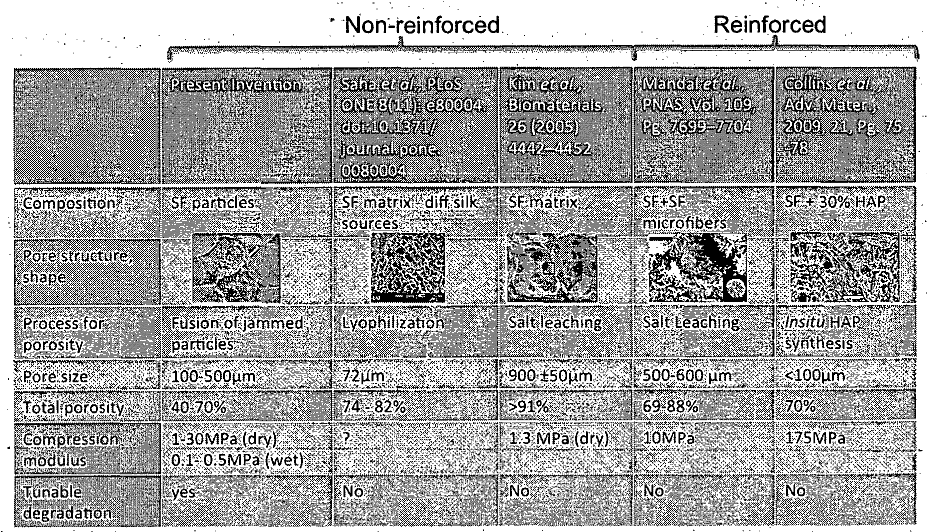

Advantages of invention:
- Does not involve use of any other organic or inorganic chemicals.
- The pore shape and size can be controlled.
- The microstructure (conformation) of the silk fibroin protein, which will assist in development of scaffolds with critical control on degradability, can be tuned.
- Also, the procedure gives the added flexibility of incorporating other biological and sensitive drugs/formulations into the particles.

## Claims

1. A process for the preparation of silk fibroin 3D scaffolds, comprising the steps of:
a) providing silk fibroin particles from regenerated silk fibroin (RSF) solution by adding RSF solution into liquid nitrogen dropwise and further freezing, lyophilizing and annealing to introduce beta sheet in the presence of a solvent by known method;
b) fusing the particles of step (a) with dilute RSF solution in a mold for compressing and placing the mold in a vacuum oven for a period ranging between 1-4 hours at temperature ranging between 40-60°C for drying to form a scaffold.

2. The process according to claim 1, wherein the particles of step (a) before freezing and lyophilizing are amorphous or semi crystalline.

3. The process according to claim 1, wherein the particles of step (a) are freezed at a temperature in the range -20 to -8 °C and lyophilized at a temperature in the range of -48 to -55°C.

4. The process according to claim 1, wherein the solvent for annealing in step (a) is selected from water or methanol.

5. The process according to claim 1, wherein concentration of dilute RSF solution used in step (b) is in the range of 1-5wt%.

6. The process according to claim 1, wherein the particle size is in the range of 0.1-3mm and the pore size in the range of 100-500µm with interconnections.

7. The process according to claim 1, wherein the scaffolds have a modulus of-1-14.7MPa) in dry state and 0.1-0.5 MPa in wet state.

8. A silk fibroin 3D scaffold prepared by the process according to claim 1, wherein said scaffold has amorphous, partially crystalline or crystalline microstructure comprising regenerated silk fibroin having porous silk fibroin particles in the particle range of 0.1-3 mm and intra particle pore size in the range of 1 - 20µm, optionally loaded with additives, wherein said scaffold has a modulus of 1-14.7MPa in dry state and 0.1 - 0.5 MPa in wet state and the pore size in the range of 100-500µm with interconnections and porosity in the range of 40-70 %.

9. The silk fibroin scaffold according to claim 8 for use in the incorporation of drug, protein, growth factor or a filler for biomedical application.

10. The silk fibroin scaffold according to claim 8, wherein degradation of the scaffold is turned by controlling the conformation of silk fibroin protein.

## Patentansprüche

1. Ein Verfahren zur Herstellung von Seidenfibroin-3D-Gerüststrukturen, umfassend die Schritte von:
a) Bereitstellen von Seidenfibroin-Partikeln aus regenerierter Seidenfibroin (RSF)-Lösung durch tropfenweise Hinzufügung einer RSF-Lösung in flüssigen Stickstoff und weiterhin Gefrieren, Lyophilisieren und Aufschmelzen, um ein Beta-Faltblatt einzuführen, in Gegenwart eines Lösungsmittels durch ein bekanntes Verfahren;
b) Zusammenführen/Vereinigen der Partikel gemäß Schritt (a) mit verdünnter RSF-Lösung in einer Gussform zum Komprimieren und Positionieren der Gussform in einem Vakuumofen für eine Dauer von zwischen 1 und 4 Stunden bei einer Temperatur von zwischen 40-60°C zum Trocknen, um eine Gerüststruktur zu bilden.

2. Das Verfahren nach Anspruch 1, wobei die Partikel gemäß Schritt (a) vor dem Gefrieren und Lyophilisieren amorph oder semi-kristallin sind.

3. Das Verfahren nach Anspruch 1, wobei die Partikel gemäß Schritt (a) bei einer Temperatur im Bereich von -20 bis -80°C gefroren werden und bei einer Temperatur im Bereich von -48 bis -55°C lyophilisiert werden.

4. Das Verfahren nach Anspruch 1, wobei das Lösungsmittel für das Aufschmelzen gemäß Schritt (a) ausgewählt ist aus Wasser oder Methanol.

5. Das Verfahren nach Anspruch 1, wobei die Konzentration von verdünnter RSF-Lösung, die gemäß Schritt (b) verwendet wird, im Bereich von 1 bis 5 Gewichtsprozent liegt.

6. Das Verfahren nach Anspruch 1, wobei die Partikelgröße im Bereich von 0,1 bis 3 mm ist und die Porengröße im Bereich von 100 bis 500 µm mit gegenseitigen Verbindungen ist.

7. Das Verfahren nach Anspruch 1, wobei die Gerüststrukturen ein Modul von 1-14,7 MPa im Trockenzustand und 0,1-0,5 MPa im Nasszustand haben.

8. Die Seidenfibroin-3D-Gerüststruktur, wie durch das Verfahren gemäß Anspruch 1 hergestellt, wobei diese Gerüststruktur eine amorphe, teilweise kristalline oder kristalline Mikrostruktur aufweist, die regeneriertes Seidenfibroin mit porösen Seidenfibroin-Partikeln in einer Partikelgröße im Bereich von 0,1-3 mm und mit einer Intrapartikel-Porengröße im Bereich von 1-20 µm umfasst, optional mit Additiven beladen, wobei diese Gerüststruktur ein Modul von 1-14,7 MPa im Trockenzustand und 0,1-0,5 MPa im Nasszustand hat, und die Porengröße im Bereich von 100-500 µm mit gegenseitigen Verbindungen und die Porosität im Bereich von 40-70% liegt.

9. Die Seidenfibroin-Gerüststruktur nach Anspruch 8 zur Verwendung für die Einarbeitung eines Wirkstoffs, Proteins, Wachstumsfaktors oder Füllstoffs für biomedizinische Anwendung.

10. Die Seidenfibroin-Gerüststruktur nach Anspruch 8, wobei der Abbau der Gerüststruktur durch die Kontrolle der Konformation des Seidenfibroin-Proteins eingestellt ist.

## Revendications

1. Procédé de préparation de supports 3D en fibroïne de soie, comprenant les étapes suivantes :
a) la fourniture de particules de fibroïne de soie à partir d'une solution de fibroïne de soie régénérée (RSF) en ajoutant goutte-à-goutte une solution de RSF à de l'azote liquide et en effectuant ensuite une congélation, une lyophilisation et un recuit pour introduire un feuillet bêta en présence d'un solvant par un procédé connu ;
b) la fusion des particules de l'étape (a) avec une solution diluée de RSF dans un moule à des fins de compression et le placement du moule dans une étuve à vide pendant une période située entre 1 à 4 heures à une température située entre 40 et 60 °C à des fins de séchage pour former un support.

2. Procédé selon la revendication 1, dans lequel les particules de l'étape (a) avant congélation et lyophilisation sont amorphes ou semi-cristallines.

3. Procédé selon la revendication 1, dans lequel les particules de l'étape (a) sont congelées à une température située dans la plage allant de -20 à -80 °C et lyophilisées à une température située dans la plage allant de -48 à -55 °C.

4. Procédé selon la revendication 1, dans lequel le solvant pour le recuit dans l'étape (a) est choisi parmi l'eau ou le méthanol.

5. Procédé selon la revendication 1, dans lequel la concentration de la solution diluée de RSF utilisée dans l'étape (b) est située dans la plage allant de 1 à 5 % en poids.

6. Procédé selon la revendication 1, dans lequel la taille des particules est située dans la plage allant de 0,1 à 3 mm et la taille des pores est située dans la plage allant de 100 à 500 µm avec des interconnexions.

7. Procédé selon la revendication 1, dans lequel les supports présentent un module de 1 à 14,7 MPa à l'état sec et de 0,1 à 0,5 MPa à l'état humide.

8. Support 3D en fibroïne de soie préparé par le procédé selon la revendication 1, dans lequel ledit support possède une microstructure amorphe, partiellement cristalline ou cristalline comprenant une fibroïne de soie régénérée ayant des particules poreuses de fibroïne de soie dans la plage de particule de 0,1 à 3 mm et une taille de pore intraparticule située dans la plage allant de 1 à 20 µm, facultativement chargé avec des additifs, dans lequel ledit support présente un module de 1 à 14,7 MPa à l'état sec et de 0,1 à 0,5 MPa à l'état humide et la taille des pores est située dans la plage allant de 100 à 500 µm avec des interconnexions et une porosité située dans la plage allant de 40 à 70 %.

9. Support en fibroïne de soie selon la revendication 8 pour son utilisation dans l'incorporation d'un médicament, d'une protéine, d'un facteur de croissance ou d'une matière de charge pour une application biomédicale.

10. Support en fibroïne de soie selon la revendication 8, dans lequel la dégradation du support est ajustée en contrôlant la conformation de la protéine de fibroïne de soie.
